# NEUE EUROPÄISCHE PATENTSCHRIFT

(11) **EP 0 788 613 B2**
(45) Veröffentlichungstag und Bekanntmachung des Hinweises auf die Entscheidung über den Einspruch: **04.06.2008**
(45) Hinweis auf die Patenterteilung: 09.12.1998
(21) Anmeldenummer: 95936543.8
(22) Anmeldetag: 25.10.1995
(51) Int. Cl.: G02B 21/00, A61B 19/00

(54) **MIKROSKOP, INSBESONDERE OPERATIONSMIKROSKOP**
MICROSCOPE, IN PARTICULAR FOR SURGICAL OPERATIONS
MICROSCOPE, NOTAMMENT MICROSCOPE UTILISE EN CHIRURGIE

(30) Priorität: 26.10.1994 CH 321794
(43) Veröffentlichungstag der Anmeldung: 13.08.1997
(73) Patentinhaber: Leica Microsystems (Schweiz) AG, 9435 Heerbrugg (CH)
(72) Erfinder: PENSEL, Jürgen, CH-9453 Eichberg (CH); STÜTTLER, Herbert, M., A-6830 Rankweil (AT)
(74) Vertreter: Rosenich, Paul
(86) Internationale Anmeldenummer: PCT/EP1995/004178
(87) Internationale Veröffentlichungsnummer: WO 1996/013743

(56) Entgegenhaltungen:
- WO-A-93/14454
- GB-A- 2 272 124
- US-A- 4 202 037
- US-A- 4 722 056
- US-A- 4 973 149
- FUNKSCHAU, Bd. 5, 1990 DE, Seiten 66-67, K.FELLBAUM ET AL. 'Sprache-elektronisch im Griff'
- PATENT ABSTRACTS OF JAPAN vol. 17 no. 464 (P-1599) ,24.August 1993 & JP,A,05 108252 (NEC CORP) 30.April 1993,
- INFORMATIQUE 92; PROCEEDINGS : INTERATIONAL CONFERENCE INTERFACE TO REAL AND VIRTUAL WORLDS., 27.März 1992 NANTERRE, FR, Seite 659 J.CHARLIER ET AL. 'COMMANDE PAR LE REGARD D'UN SYSTEME DE VISUALISATION 2D: EXEMPLE DU MICROSCOPE OPERATOIRE'

## Beschreibung

Die Erfindung betrifft ein Mikroskop, insbesondere ein Operationsmikroskop.

Der Stand der Technik kennt zwei Hauptgruppen von Mikroskopen:
1) eintubige monokulare Mikroskope und
2) zweitubige Stereomikroskope.

Verstellorgane für die optischen Bauteile der Mikroskope und/oder für Arbeitstische sind bei beiden Varianten bekannt.

Da sich der Anwender in der Regel der Beobachtung des durch das Mikroskop betrachteten Objektes widmet, müssen solche Verstellorgane im Bereich zwischen Objektiv und Okular untergebracht sein, damit der Anwender sie gut erreichen kann.

Bei verschiedenen Anwendungen, insbesondere jedoch bei Operationsmikroskopen, ist es für einen Anwender (Operateur) schwierig, das Mikroskop derart zu bedienen, da er in der Regel beide Hände benötigt, um die Operation bzw. die Manipulation am Objekt durchführen zu können. Um den Anwendern in solchen Situationen entgegenzukommen, wurden elektromotorische Stellorgane entwickelt, die über eine Fernbedienung (elektrische Schalter) die gewünschten Ein-bzw. Verstellungen vornehmen. Um dabei die Hände des Anwenders völlig zu entlasten, wurden dem Mikroskop Fussschalter zur Seite gestellt, über die der Anwender verschiedene, begrenzte Einstellungen, wie z.B. von Zoom, Fokus usw. vornehmen konnte.

Gerade bei Operationen ist es für einen Anwender, der auf ein Blickfeld und seine Hände konzentriert ist, jedoch nicht leicht, zusätzliche Bewegungen der Füsse zur Betätigung von unterschiedlichen Fussschaltern o.dgl. zu koordinieren. Deshalb wurden im Zuge von aufwendigen Weiterentwicklungen sprachgesteuerte Stellorgane geschaffen, die vom Anwender mittels Sprache bedient wurden. Spracherkennungssoftware ist jedoch noch nicht fehlerfrei und insbesondere unter Operationsstress läuft man Gefahr, dass es durch eine Befehlsmissdeutung zu einer Fehlbedienung des Mikroskops kommt, was bei bestimmten schwierigen Operationen für den Patienten katastrophale Folgen haben kann. Zudem ist Spracherkennungssoftware in der Regel nicht geeignet, unterschiedliche Anwender zu verstehen, bzw. müssen durch aufwendige Spracheinstudierungsübungen alle Anwender ihr Sprachprofil abspeichern, was einigen Aufwand bedeutet und die Einsatz fähigkeit des Mikroskops reduziert. Auch Mundschalter wurden entwickelt, die jedoch erstens unangenehm sind und zweitens bei notwendigem Mundschutz kaum angewendet werden können.

Selbstverständlich sind diverse Eye-Tracking Methoden bekannt. Wenigstens bereits seit Anfang 1994 ist die erwähnte Kamera der Firma Canon für das Autofocussiern bekannt. Ein französischer Bericht "Informatique 92" hat mit Stand 1992 ein fertiges System entwickelt, bei dem ein Mikroskop mittels Eye-Tracking so gesteuert wird, dass wenn eine Operateur seine Blickrichtung durch den Tubus ändert (er blickt z.B. nach links) dass Mikroskop seine Lage im Raum ändert und die Tuben auf die neue Blickrichtung des Operateurs orientiert bzw. schwenkt. Dieses Verfahren wird in der Praxis nicht angewendet, da es häufig vom Operateur gar nicht gewünscht ist, das bisher Gesehene gegebenenfalls aus dem Blickwinkel zu verlieren. Darüber hinaus führt die automatische Verstellung des Mikroskopes entsprechend dieser bekannten Lehre dazu, dass das ursprünglich optisch justierte Mikroskop nun im Zentrum seiner Blickrichtung plötzlich unjustiert sein kann, da sich in der Regel durch das Verschwenken der Tuben auch der Z-Abstand ändert. Lediglich bei geringen Vergrösserungen oder bei Mikroskopen mit eingebautem externen Autofocus (nicht augenorientiert) könnte dieses System gegebenenfalls praktisch angewendet werden.

Eine japanische Publikation JP-A-51108252 bezieht sich mit Stand 1993 auf einen augengesteuerten Cursor in einem Computer. Bei der Betrachtung eines Computermonitors wird ein Anwender überwacht, so dass seine Augenbewegungen das Geschehen am Bildschirm steuern können. Dieser Stand der Technik liegt jedoch abseits der Mikroskopie und es ergibt sich daraus auch kein Hinweis auf die erfindungsgemässe Lehre. Das Steuern von Mikroskopantrieben o.dgl. ist nicht nahegelegt. Ähnliches ergibt sich aus dem US-Patent 4973149, das bereits früher vorgeschlagen hatte, Anwenderaugen bei der Betrachtung eines Computermonitors zu überwachen und dem Anwender so die Möglichkeit zu geben, den Computer oder seine Umgebung zu steuern. Die WO-A-93/14454 bezieht sich ebenso mit Stand 1993 auf die Ansteuerung eines Computermonitors mittels Eye-Tracking.

Aus DE-A-4 018 400 ist ein Mikroskop für einen Anwender bekannt, mit wenigstens einem Tubus, wenigstens einem Bedienorgan, wenigstens einem ferngesteuerten Stellorgan und wenigstens einer Steuerung für dieses Stellorgan und mit einem Sensor zur Erfassung der Augen- bzw. Pupillenstellung eines Betrachterauges für die Steuerung des Stellorgans, wobei das Stellorgan insbesondere ein Autozoom und einen Antrieb für ein Zoom umfaßt.

Der Erfindung liegt somit, ausgehend vom erwähnten Stand der Technik und den damit verbundenen Nachteilen, die Aufgabe zugrunde, die erwähnten Probleme zu beseitigen und ein entsprechendes fernsteuerbares Mikroskop für Anwender, deren Hände nicht durch die Mikroskopbedienung belastet werden sollen, und insbesondere geeignete Bedienorgane für das Mikroskop zu schaffen. Das neue Mikroskop soll völlige Bewegungsfreiheit für die Hände sicherstellen und auf eine Mehrfachbelastung der Füsse eines Anwenders verzichten. Weiters soll eine gute Integrierbarkeit des neuen Mikroskops in computerunterstützte Systeme mit verschiedenen zusammenarbeitenden Modulen, wie z.B. bei der Videostereomikroskopie, Videostereotaxie usw. und anderen Anwendungen, bei denen wenigstens eine fernsteuerbare oder automatische Funktion (z.B. Autofokus oder Autozoom) vorgesehen ist, gegeben sein. Die neuen Bedienorgane sollen klein bauen und im Mikroskop gut integrierbar sein.

Gelöst wird diese Aufgabe beispielhaft erstmals durch Anwendung der Merkmale des Anspruches 1, wobei unter Sensor jede Einrichtung zu verstehen ist, die eine Augenbewegung, oder einen Nervenbefehl zu einer Augenbewegung erfassen kann und daraus die durchgeführte oder geplante Augenbewegung ableiten kann.

Bevorzugt ist der Einbau eines Augen- oder Pupillenpositionserkennungsgerätes in wenigstens einen Tubus des Mikroskops. Dieses Erkennungsgerät ist erfindungsgemäss mit einer elektronischen Auswerteeinheit gekoppelt, die aufgrund einer bestimmten Pupillenstellung eines Anwenders, der durch das Mikroskop blickt, die Pupillenstellung (Position) erkennt und daraus auf bestimmte Verstellbefehle des Anwenders rückschliesst und diese an die elektromechanischen Stellorgane weitergibt.

Im Rahmen der Erfindung liegen auch Varianten mit je einem Pupillenerkennungsgerät pro Tubus. Um für Anwender mit Augenfehlstellungen jedoch Missverständnisse bei der Erkennung zu vermeiden, ist es bevorzugt, jeweils nur ein Erkennungsgerät zu benutzen. Bevorzugt könnte dies eines von zwei vorgesehenen sein - je nach Wahl des Anwenders. Empfohlen wird die Einstellung auf das Führungsauge des jeweiligen Anwenders.

Dem Anwender ist es somit möglich, sehr schnell (Augenbewegungen zählen zu den schnellsten Bewegungsmöglichkeiten eines Menschen) und eindeutig Befehle zu erteilen, indem er beispielsweise Markierungen im Tubus oder in den Tubus eingespiegelte Bilder, Symbole o.dgl. fixiert.

Wenn ein erfindungsgemässes Mikroskop innerhalb eines computerunterstützten Systems integriert ist, das z.B. noch weitere fernsteuerbare Geräte, Bildliefervorrichtungen, Operationswerkzeuge etc. umfasst, so können bei Bedarf für alle Funktionen dieser Geräte Markierungen, Symbole etc. vorgesehen sein, so dass der Anwender vergleichbar wie auf einer bildschirmaktiven (benutzeraktiven) Computermonitoroberfläche Steuerungsbefehle erteilen kann. Er benutzt dabei anstelle einer Computermaus lediglich seine Blicke, um die entsprechenden Symbole zu fixieren. Das Aktivieren der entsprechenden Symbole bzw. Befehle kann dabei dadurch erfolgen, dass der Anwender eine längere Zeit das betreffende Symbol betrachtet, oder dass er innerhalb einer bestimmten Zeit zwischen zwei Symbolen hin- und herschaut. Um dem Anwender zu zeigen, dass sein Befehl registriert wurde, kann dann beispielsweise das Symbol seine Farbe wechseln, es kann zu blinken beginnen oder durch ein anderes Symbol ersetzt werden; auch akustische Anzeigemöglichkeiten liegen im Bereich der Erfindung.

Im Rahmen der Erfindung liegen jedoch auch Kombinationen mit schon bekannten Fernsteuermechanismen, z.B. mit einem Fussschalter, der im Unterschied zum Bekannten nur mehr eine Funktion hat, z.B. die des "Anklickens" bzw. Aktivierens eines entsprechenden Symbols. Auch vereinfachte Sprachsteuerungen, die z.B. lediglich einen Laut des Anwenders erfordern, während dieser ein bestimmtes Symbol fixiert, liegen im Rahmen der Erfindung.

Um die zu betrachtenden Steuer-Symbole ins Gesichtsfeld des Anwenders zu bringen, sind verschiedene Methoden denkbar, zu denen unter anderem zählen: im Strahlengang eingesetzte Glasscheiben o.dgl. mit geeigneten Markierungen (wie z.B. Fadenkreuze mit peripheren Markierungen o.dgl.), z.B. durch LED's erregte Leuchtpunkte an der Tubusinnenseite, z.B. über Strahlenteiler eingeblendete LCD- Bildschirme usw. Dabei kann es vorteilhaft sein, Eichroutinen vorzusehen, bei denen zu Beginn der Anwendung der Betrachter auf eine bestimmte Stelle, z.B. auf ein Fadenkreuz im Tubus blickt und hernach z.B. mittels Schalter oder selbsttätig eine Eichroutine auslöst, bei der die Steuerung ermittelt, welcher Pupillenstellung der Blick auf die bestimmte Stelle bzw. das Fadenkreuz entspricht, um so davon abweichende Stellungen besser auswerten zu können. Alternativ ist es möglich, die bestimmte Stelle, z.B. das Fadenkreuz, auch auf einem geeigneten Träger verschiebbar zu gestalten, um bei willkürlich gewählter - z.B. für den Anmelder optimaler - Augenstellung die Blickrichtung mit der bestimmten Stelle in Deckung zu bringen, um bei den Steuerbefehlen durch Verschwenken der Augen aus dieser Stellung heraus eine geeignete Referenz zu haben. Die für das Auge sichtbaren Befehlsfelder werden dabei z.B. an der dem Auge zugewandten Displayoberfläche nachgeführt. Zur Referenzfindung am Beginn einer Benutzung können auch andere Verfahren dienen, sofern sie geeignet sind, eine Relativbewegung eines Auges aus einer bestimmten Position heraus festzustellen.

Um die Pupillenposition zu erfassen, liegen im Rahmen der Erfindung ebenso verschiedene Varianten, von denen jene bevorzugt ist, bei der ein auf das Auge gerichteter Messstrahl eines IR-Lasers mittels CCD optisch von der Augenoberfläche wieder abgetastet wird, um derart die Augen- bzw. Pupillenposition zu erfassen. Diesbezüglich wird auf das in einem anderen Fachgebiet - der Videotechnik - an sich bekannte "Eye control"-verfahren verwiesen, wie es von der Firma Canon beispielsweise unter der Markenbezeichnung UC-XlHi auf den Markt gebracht wird. Die durch die dort vorgesehene Einrichtung zur Erfassung der Augenstellung gewonnenen Daten werden dort u.a. verwendet, um eine Autofokusfunktion des Kameraobjektivs auf ein bestimmtes Gebiet im Gesichtsfeld des Kameramannes abzustellen. Selbstverständlich liegen im Rahmen dieser Anmeldung auch solche Varianten, bei denen ein Autofokussystem eines Mikroskops mit den optischen Bedienorganen bzw. Symbolen und der damit verbundenen Steuerung derart zusammenwirkt, dass der Autofokus dem Blick des Anwenders folgt, um das Mikroskop jeweils auf das eingeschränkte Detailgebiet scharf zu stellen, auf das sich der Anwender gerade konzentriert. Alternativ zu der optischen Augenbewegungserfassung liegen auch alle jene Verfahren im Rahmen der Erfindung, bei denen an anderen Körperstellen eines Anwenders Bewegungen oder Nervenimpulse für Bewegungsbefehle abgenommen und ausgewertet werden. So können beispielsweise auch die Bewegungen der Augenmuskulatur z.B. über Elektroden o.dgl. abgegriffen werden, um die Blickposition des Auges zu erfassen.

Besondere Vorteile liefert eine Variante, bei der Bedienorgan-Symbole im Blickfeld des Anwenders frei programmierbare Funktionen erlauben. Hiebei ergibt sich eine besonders günstige Kombination mit der als "Leica Box" am Markt bekannten Einrichtung, die verschiedenste Daten aus verschiedensten Geräten, Messeinrichtungen und elektronischen Speichern usw. für eine Bildverarbeitung und rasche Datenauswertung verarbeitbar machen. Diesbezüglich wird auf die CH-Anmeldung der Anmelderin mit dem Aktenzeichen 01091/94-3, das ist eine Prioritätsanmeldung zu der publizierten Int.Patentanmeldung WO95/27917, verwiesen, deren Lehren als im Rahmen dieser Anmeldung liegend, zum Zwecke einer möglichen und sinnvollen Kombination der beiden Lehren geoffenbart gelten.

Für mehrere unterschiedliche Anwender ist es von Vorteil, wenn einmal getroffene Einstellungen abgespeichert und bei Bedarf aufgerufen werden, so dass nach dem Anwählen einer für einen bestimmten Anwender abgespeicherten Einstellung ohne weiteren Eich- oder Justieraufwand sofort mikroskopiert werden kann.

Weiters wird auf die Lehren der CH-Anmeldung der Anmelderin mit dem Aktenzeichen 01092/94-5, das ist eine Prioritätsanmeldung zu der publizierten Int.Patentanmeldung WO95/27918, verwiesen, da die dort geoffenbarten kleinen Einblendelemente optimal bei dem erfindungsgemässen Mikroskop optimal zum Einsatz gelangen können. Im Zusammenhang mit der gegebenenfalls erforderlichen Abstandsmessung zwischen Hauptobjektiv und Objekt bzw. betrachtetes Objektdetail wird auf die Lehre der CH-Anmeldung der Anmelderin mit dem Aktenzeichen 01090/94-1, das ist eine Prioritätsanmeldung zu der publizierten Int.Patentanmeldung WO95/27917, verwiesen, die zum Zwecke der Darstellung einer weiteren Ausführungsvariante der Erfindung als hierin geoffenbart gilt. Das betrifft jeweils insbesondere die Figuren und die dazugehörigen Beschreibungsteile. Für die Schärfenmessung im vom Auge anvisierten Bereich des Objektes kann beispielsweise auch eine Bildsignalverarbeitung eingesetzt sein, die z.B. die Randschärfe von bestimmten Konturen misst und daraus auf die Abbildungsschärfe rückschliesst und entsprechende Stellorgane ansteuert, um die Abbildungsschärfe zu verbessern.

Um im Falle der Beleuchtung des Anwenderauges mittels IR-Diode eine Schonung der Netzhaut vor übermässiger IR-Strahlung zu erreichen, ist gemäss einer Weiterbildung der Erfindung die IR-Diode im Takt der CCD-Ausleserate gepulst, wobei die CCD-Ausleserate vorzugsweise frei wählbar ist.

Weitere Details und Merkmale der Erfindung sind in den Patentansprüchen offengelegt und gekennzeichnet. Insofern ist die vorgängige Darstellung nicht erfindungsbeschränkend. Die Offenbarung aller Varianten und Ausbildungsformen ergibt sich aus dem Gesamttext der Anmeldung, die die Ansprüche einschliessen

Anhand eines beispielhaften, mittels Skizzen dargestellten Ausführungsbeispieles wird die Erfindung beispielhaft näher erläutert. Es zeigen dabei:
- Fig. 1: ein Blockschaltbild mit einer Auswahl von möglichen Funktionen und Einrichtungen, die mittels der erfindungsgemässen optischen Bedienorgane ferngesteuert werden können.
- Fig.2: eine symbolische Anordnung, bei der die Symbole von einem LCD über einen Strahlenteiler in den Okularstrahlengang eingeblendet werden
- Fig.3: ein symbolischer Aufbau eines Operationsmikroskopes mit erleichterter Anwendung und erfindungsgemässen Möglichkeiten und
- Fig.4: ein Detail eines Mikroskopes, bei dem eine einzige Bildaufnahmevorrichtung sowohl der Pupillenstellungs erfassung als auch der Aufnahme des Bildes vom Objekt dient.

Die Figuren werden zusammenhängend beschrieben. Gleiche Bezugszeichen bedeuten gleiche Bauteile. Funktionsähnliche Bauteile tragen gleiche Bezugszeichen mit unterschiedlichen Indizes.

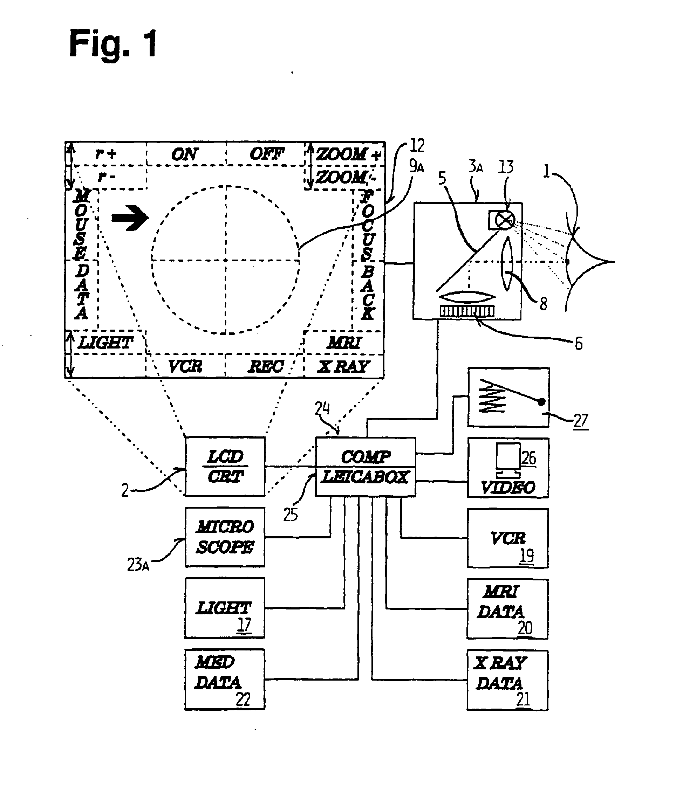

Fig.1 zeigt mit 12 etwa das Bild, das sich einem Betrachter ergibt, der durch ein Okular in einen Tubus eines erfindungsgemässen Mikroskops 23 blickt. Was hier im Bild nicht zu sehen ist, ist das Objekt, das gegebenenfalls auch zu sehen wäre; es ist dem Anzeigenfeld des Bildes 12 optisch unterlegt. Bei den dargestellten Beispielen ist es nicht wesentlich, ob der Blick auf das Objekt rein durch optische Bauteile ermöglicht wird, oder ob eventuell auch eine Videosignalverarbeitung zwischengeschaltet ist, wie z.B. bei einem Video-Endoskop, das ebenso im Rahmen dieser Erfindung liegt.

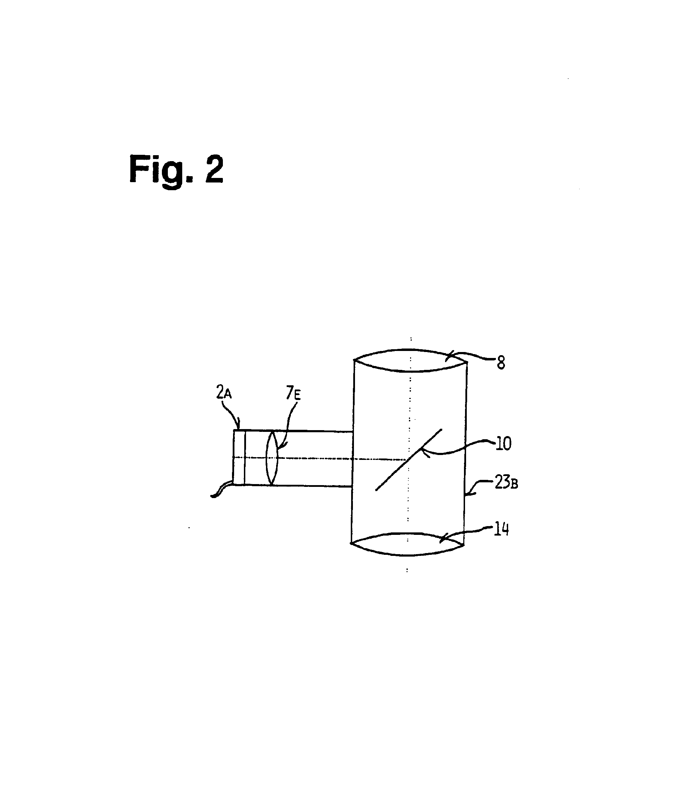

Fig.2 zeigt, wie das Bild 12 in den Tubusstrahlengang des Mikroskops 23b eingespiegelt wird. Ein LCD- oder CRT- Display 2a wirft über eine Optik 7e und einen Teilerspiegel 10 das durch eine Datenverarbeitung 24 generierte Bild durch eine Okularlinse 8 in ein Betrachterauge 1. In Fig. 1 ist ein solches LCD oder CRT mit 2 bezeichnet. Im Falle eines Videoendoskops oder eines Operationsmikroskops mit einem LCD-oder CRT-Display als Objektwiedergabeeinheit, kann die separate optische Einblendung eines Bildes entfallen, wenn dieses auf elektronischem Weg mit dem Bild des Objektes gekoppelt wird.

Fig.1 zeigt rechts neben dem Bild 12 symbolisch eine Pupillenpositionserkennungseinrichtung 3a, die skizzenhaft angedeutet die Okuklarlinse 8 sowie einen teildurchlässigen Spiegel 5 - der vorzugsweise nur für infrarotes Licht spiegelnd wirkt - umfasst, über den ein CCD 6 o.dgl. die Position der Pupille eines Betrachterauges 1 abtastet. Die Pupille wird dabei von einer IR-LED 13 beleuchtet, so dass die geometrische Form der Pupille deutlich am CCD 6 abgebildet werden kann. Mittels nicht näher angedeuteter Bildverarbeitung z.B. in der Datenverarbeitung 24 wird die derart abgetastete Pupillenpositionsinformation ausgewertet und dadurch festgestellt, wohin das Auge im Bild 12 blickt. Derart kann ermittelt werden, auf welches der in 12 strichliert eingezeichneten Felder das Auge blickt, um daraus die entsprechend gewünschten Befehle ableiten zu können. In der gezeigten Variante ist auch ein Fussschalter 27 vorgesehen, mittels dem eine zuvor mit dem Auge ausgewählte Funktion bestätigt "angeklickt" wird.

Eine Auswahl möglicher Befehle und Funktionen wird beispielhaft und unvollständig aufgezählt: Ein/Ausschalten des Befehlsfeldes 12; für das Mikroskop 23a kann die Vergrösserung gewählt (Gamma) und die Zoomeinstellung verändert werden. Ein automatisches Fokustracking kann so erfolgen, dass die Pupillenpositionserkennungsvorrichtung 3 stets ermittelt, auf welche Stelle am Objekt das Auge blickt, um daraufhin das Mikroskop 23a auf diese Stelle zu fokussieren; Die Steuerung einer Videokamera 26, die an das Mikroskop angeschlossen ist; Steuerung eines Videorekorders 19 ; z.B. auch mit einer Kurzwiederholfunktion, bei der die letzten Sekunden der vorgänge am Objekt wieder auf dem LCD oder CRT dargestellt werden, um dem Anwender eine Orientierungshilfe zu geben; eine Überlagerung des Bildes des Objektes mit einem positionsgleichen Bild einer MRI-Aufnahme 20 oder einer Röntgenaufnahme 21 o.dgl.; eine Steuerung der Beleuchtung 17 des Mikroskops 23a; das Überlagern von patientenbezogenen Daten (Med Data) 22, wie z.B. Blutdruck, EKG, Puls usw; abgesehen von den beschriebenen Funktionen kann auch noch eine bewegliche "Maus" als Cursor oberflächenaktiv abgebildet sein, die durch Augenbewegung gesteuert werden kann, wie dies mit einer Maus bei einem Computerbildschirm möglich ist. Das Positionieren der "Maus" in einem Teil des Bildes mit anschliessender Bestätigung durch den Fussschalter kann entsprechend auch die beschriebenen oder andere ergänzende Funktionen auslösen, wie z.B. das Markieren einer bestimmten Stelle für sofortige oder spätere Bilddatenverarbeitung, für das Zeichnen von Konturlinien usw.

In der Regel werden alle Funktionen und Steuerungen der beschriebenen Geräte von der Datenverarbeitung gesteuert. Zur schnelleren Datenverarbeitung ist dieser Datenverarbeitung 24 eine Datenaufbereitungseinheit 25 vorgeschaltet, die alle, zum Teil unterschiedlichen Datenformate auf ein gemeinsam verarbeitbares Format umwandelt bzw. Befehlsdaten in geeignete Gerätesteuerungsdaten umwandelt.

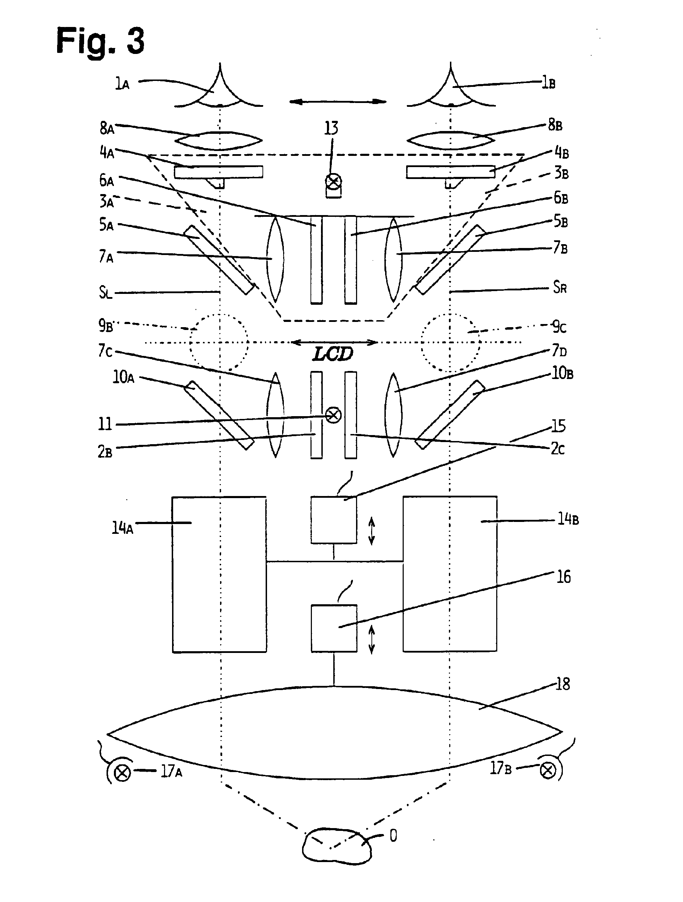

Fig.3 zeigt schematisch ein Hauptobjektiv 18, das selbst, wie angedeutet, oder dessen verstellbare Optik, wie nicht gezeigt, durch einen Fokuseinstellungsantrieb 16 steuerbar ist und eine Mikroskopbeleuchtung 17. Weiters ist ein Zoom 14 mit entsprechenden Zoomantrieb 15 vorgesehen.

Teilerspiegel 10 dienen dem Einspiegeln von Bildinformationen aus zwei LCD'S 2a,b, die je einem der beiden Strahlengänge zugeordnet sind. Die LCD'S geben das Bild 12 und allfällige MRI-,Röntgen- oder Patientendaten wieder. Hinter den LCD's ist eine Hintergrundbeleuchtung 11 angeordnet. Optiken 7c und d dienen einer guten Abbildung. Symbolisch und um 90° in die Bildebene gedreht sind Fadenkreuze 9b und c dargestellt, die z.B. auf verschiebbaren Glasplatten in jeden Strahlengang eingeschoben sind und Eichzwecken dienen.

Teiltransparente IR-Spiegel 5a,b erlauben einen freien Durchblick im sichtbaren Bereich auf das Objekt. IR-Strahlen werden hingegen über Optiken 7a und b auf CCD'S 6a und b abgebildet, wobei die Optiken 7a und b so ausgelegt sind, dass auf den CCD'S die Pupille eines Betrachterauges 1 abgebildet wird. Vorzugsweise wird jedoch wahlweise stets nur ein Auge berücksichtigt, um im Falle von Augenfehlstellungen o.dgl. Missverständnisse bei der Auswertung zu vermeiden.

Die Augen werden durch die Okularlinsen 8a und b und/oder unabhängig davon von einer IR-LED 13 beleuchtet, um die erwähnte Abbildung am CCD 6 zu ermöglichen. Im Falle der Beleuchtung durch die Okularlinsen 8,8b sind diesen kleine Einblendelemente 4a bzw. b vorgeschaltet, die über kleine reflektierende Flächen IR-Beleuchtung den Betrachteraugen zuführen. Sofern das zur Beleuchtung verwendete Licht genügend gestreut ist, ist es unkritisch, an welchem Ort die kleine reflektierende Fläche angebracht ist. Bei einer konzentrischen, gering streuenden Beleuchtung mittels Lichtkegel auf das Zentrum der Augapfeloberfläche wäre es bevorzugt, die kleine reflektierende Fläche möglichst axial im Strahlengang anzuordnen.

Der Gegenstand der Fig.3 zeigt einen Aspekt der Erfindung insbesondere für Stereomikroskope. Da zwei Pupillenpositionserkennungsgeräte 31,3b vorgesehen sind, die Pupillenpositionen unabhängig voneinander auf je einem CCD 6a oder 6b abbilden, ermöglicht dieser Aufbau die automatische Einstellung des Augenabstandes mittels eines nicht näher dargestellten Antrieb, der in Abhängigkeit vom gemessenen Abstand der Pupillen aus der Strahlengangmitte, die beiden Tuben so zueinander bewegt, dass beide Pupillen optimale Sicht durch das Mikroskop haben. Ein derartig ausgerüstetes Mikroskop arbeitet praktisch automatisch und es genügt, wenn ein Betrachter durchblickt, um die wichtigsten nötigen Einstellungen zu erlangen. Sollten diese für einen bestimmten Betrachter schon vorbestimmt und abgespeichert sein, genügt die Angabe des betreffenden Anwenders z.B. mittels Eingabe eines Codes in ein Bedienfeld, wie Tastatur oder mittels Eingabe über einen Wahlschalter usw.

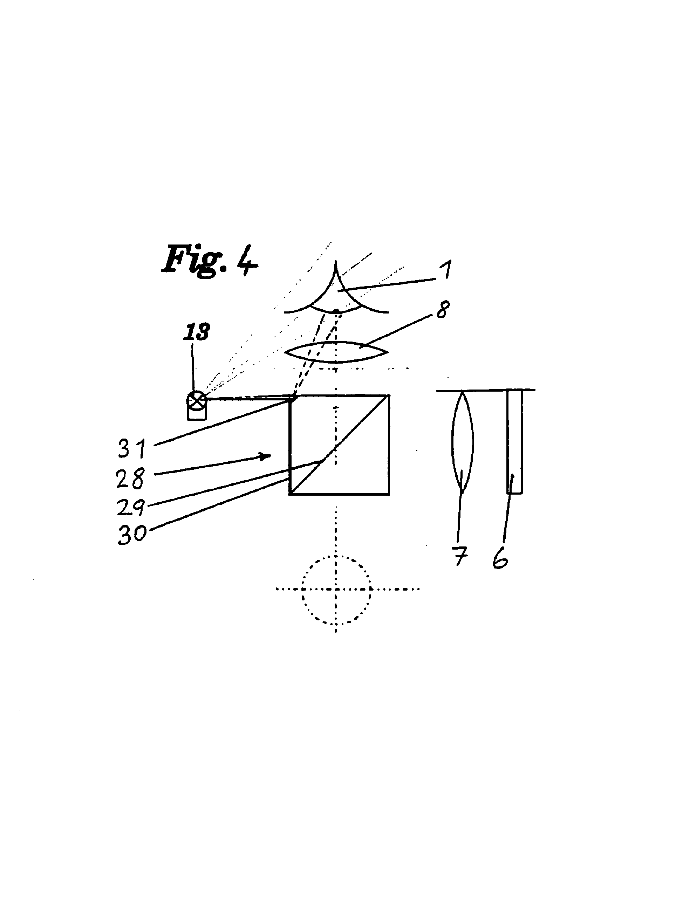

Nicht näher eingezeichnet, weil im Stand der Technik bekannt, sind Ausspiegelvorrichtungen für eine Videokamera o.dgl. Jedoch zeigt Fig.4 einen im Rahmen der Erfindung, jedoch auch unabhängig von dieser mit Vorteil einsetzbaren Teilerwürfel 28, an dessen Teilerfläche 29 das betrachtete Bild des Objekts zum Teil seitlich durch eine Optik 7 auf ein Kamera-CCD 6 geworfen wird, während der andere Teil dem Auge 1 zur Verfügung steht. Gleichzeitig wird die vom Auge kommende IR-Strahlung an der Teilerfläche 29 seitlich nach links gespiegelt, um dort auf einen Spiegelfläche 30 zu treffen, von der es durch die Teilerfläche 29 und die Optik 7 ebenso auf das CCD 6 geworfen wird. Zweckmässig sind die Okularlinse und die nicht gezeigte übrige Optik im Strahlengang so abgestimmt, dass das Objekt und die Augenoberfläche genügend auf dem CCD abgebildet sind, um sodann rechnerisch aus dem überlagerten Bild die nötigen Informationen über die Pupillenstellung herauszufinden und den Eintrag der IR-Strahlung aus dem Videobild danach wieder zu löschen. Der Vorteil eines solchen Aufbaus liegt im Verzicht auf ein zweites CCD. Als Besonderheit bei dem Teilerwürfel 28 ist noch die optional vorgesehene Fase 31 zu erwähnen, die ebenso - insbesondere für IR-Strahlung reflektierend ausgebildet ist und so die Strahlen aus der IR-Diode 13 auf das Auge 1 lenkt.

Die Erfindung beschäftigt sich weiter mit einem vom bisher Gesagten auch unabhängig anwendbaren Aspekt: Im Okularbereich ist bevorzugt eine Positionierauflage für den Kopf- bzw. das Gesicht eines Anwenders vorgesehen, die durch Fixierung von Gesichtspartien des Anwenders als Horizontal- und/oder Vertikal-Referenz und/oder Entspannungshalterung dient. Eine solche Positionierauflage erfüllt dabei die Aufgabe, sicherzustellen, dass eine einmal gewählte Einstellung des Mikroskopes als richtig und verwertbar wieder eingenommen werden kann, nachdem der Anwender seinen Kopf vom Mikroskop einmal entfernt hat. Vor allem bei lang andauernden Operationen ist dies von Vorteil. Zusätzlich ergibt sich der Effekt, dass Ermüdungserscheinungen eines Operateurs durch bisherige permanente Nackenmuskelanstrengung reduziert werden.

Die Pupillenpositionserkennungseinrichtung ist selbstverständlich mit einem Schaltglied ausgestattet, dass die Messroutine und folglich auch die Verstellroutine unterbricht sobald die Pupillenpositionserkennungseinrichtung keine Pupille ausmachen kann.

### Bezugszeichenliste

- 1a,b: Betrachterauge
- 2a,b,c: LCD oder CRT für Display im Okularstrahlengang
- 3a,b: Pupillenpositionserkennungseinrichtung
- 4a,b: Einblendelement
- 5a,b: teildurchlässiger IR-Spiegel (Reflexionsrate für IR-Strahlung gegen 100%, für übrige Strahlung gegen 0%)
- 6a,b: CCD oder funktionsähnlicher Sensor z. Pupillenlagebestimmung
- 7a,b,c,d,e: Abbildungsoptik
- 8a,b: Okularlinse
- 9a,b,c: Fadenkreuz
- 10a,b: Teilerspiegel
- 11: LCD Hintergrundbeleuchtung
- 12: beispielhaftes Bild des LCD oder CRT, wie für das Auge 1 durch das Okular 8 gesehen, dem Bild des betrachteten Objektes überlagert;
- 13: LED für IR-Licht, eventuell getaktet
- 14a,b: Zoom
- 15: Zoomantrieb
- 16: Fokuseinstellungsantrieb
- 17a,b: Beleuchtung
- 18: Hauptobjektiv
- 19: Videorekorder z. Abspielen und Aufnahmen von Videobildern;
- 20: Bildliefervorrichtung für MRI-Daten vom betrachteten Objekt;
- 21: Bildliefervorrichtung für Röntgen-Daten vom betrachteten Objekt;
- 22: Bildinformationsgenerator für patientenbezogene Informationen, z.B. wie Name, Alter, Blutdruck, Herzfunktion usw.
- 23: Mikroskop
- 24: Datenverarbeitung
- 25: Datenaufbereitung zur Datenformatvereinheitlichung (Leica Box)
- 26: Videokamera
- 27: Fussschalter
- 28: Teilerwürfel
- 29: Teilerfläche
- 30: Spiegelfläche insbesondere für IR-Strahlen
- 31: reflektierende Fase
- O: Objekt
- SL: Strahlengang (Mitte)
- SR: Strahlengang (Mitte)

## Patentansprüche

1. Mikroskop für einen Anwender, mit wenigstens einem Tubus, wenigstens einem Bedienorgan, wenigstens einem ferngesteuerten Stellorgan und wenigstens einer Steuerung für dieses Stellorgan und mit einem Sensor zur Erfassung der Augen- bzw. Pupillenstellung eines Betrachterauges für die Steuerung des Stellorgans, **dadurch gekennzeichnet, dass** das Stellorgan ein Autozoom und/oder Antriebe für ein Zoom (15), für eine Fokuseinstellung (16), für eine Augenabstandseinstellvorrichtung, für eine Beleuchtung (17) und/oder einen Videorecorder (19), eine Bildliefervorrichtung für MRI-Bilddaten (20) oder Röntgendaten (21), und/oder eine Patientendatenwiedergabeeinrichtung (22) umfasst, und dass für jedes Betrachterauge (1a,1b) eine Pupillenpositionserkennungseinrichtung (3a,3b) vorgesehen ist, die die Position jeder Pupille von der ihr zugeordneten Strahlengangmitte erkennbar macht und mit einer Augenabstandseinstellvorrichtung verbunden ist, die automatisch den Tubenabstand an den Augenabstand eines Betrachters anpasst.

2. Mikroskop nach Anspruch 1, **dadurch gekennzeichnet, dass** im Bereich des Tubus dem Auge (1) Mittel für das Darstellen von für den Anwender im Inneren des Tubus sichtbare Bedienorgansymbolen zugeordnet sind, wobei eine Erkennungseinrichtung über eine Datenverarbeitungseinrichtung (24) zur Blickdeckungserfassung mit den Bedienorgansymbolen und weiters mit dem Stellorgan bzw. mit den Antrieben für ein Zoom (15), für die Fokuseinstellung (16), für die Augenabstandseinstellvorrichtung, für die Beleuchtung (17) und/oder mit dem Videorecorder (19), der Bildliefervorrichtung für MRI-Bilddaten (20) oder Röntgendaten (21), und/oder der Patientendatenwiedergabeeinrichtung (22) verbunden ist.

3. Mikroskop nach Anspruch 1 oder 2, **dadurch gekennzeichnet, dass** wenigstens einem Auge (1) ein Display für das Einblenden von Schaltsymbolen und/oder ein Display für das für den Anwender im Inneren des Tubus sichtbar Darstellen von Bedienorgansymbolen oder der in Anspruch 2 definierten Bedienorgansymbole zugeordnet ist, wobei als Display bevorzugt für die Bedienorgansymbole sowie für allfällige MRI- oder Röntgen- oder Patientendaten ein LCD (2b oder c) zugeordnet ist, das über eine Optik (7c oder d)und über einen Teilerspiegel (10a oder b) in eine Betrachterauge (1a,1b) abbildet, wobei im Falle eines Stereomikroskops mit zwei CCD'S den beiden CCD'S vorzugsweise eine gemeinsame Hintergrundbeleuchtung (11) zugeordnet ist.

4. Mikroskop, nach einem der vorhergehenden Ansprüche, **dadurch gekennzeichnet, dass** die Augen- oder Pupillenpositionserkennungseinrichtung (3) bzw. der Sensor wenigstens eine IR-LED (13) und wenigstens ein CCD (6) zum Abtasten des Lichtes der IR-LED (13) an der Augenoberfläche umfasst, wobei das CCD (6) mit einer Auswerteschaltung bzw. mit einer Datenverarbeitungseinrichtung (24) oder der in Anspruch 2 definierten Datenverarbeitungseinrichtung verbunden ist, die das Stellorgan, bzw. die Antriebe für ein Zoom (15), für eine Fokuseinstellung (16), für eine Augenabstandseinstellvorrichtung, für eine Beleuchtung (17) und/oder einen Videorecorder (19), die Bildliefervorrichtung für MRI-Bilddaten (20) oder Röntgendaten (21), und/oder die Patientendatenwiedergabeeinrichtung (22) steuert.

5. Mikroskop nach einem der vorhergehenden Ansprüche, **dadurch gekennzeichnet, dass** bei zwei Sensoren oder zwei Pupillenpositionserkennungseinrichtungen (3a,3b) wahlweise nur eine in Betrieb nehmbar ist, und/oder dass den unterschiedlichen Funktionen Module zugeordnet sind, die bausteinförmig zusammensetzbar bzw. zu- oder abschaltbar sind.

6. Mikroskop nach einem der Ansprüche 2-5, **dadurch gekennzeichnet, dass** der Datenverarbeitungseinrichtung (24) ein Speicher für das Abspeichern und automatische Abrufen von Einstellungsdaten bzw. -vorgängen des Mikroskopes in Abhängigkeit von eingebbaren Anwender - Codes zugeordnet ist.

7. Mikroskop nach einem der Ansprüche 2 bis 6, **dadurch gekennzeichnet, dass** die Pupillenpositionserkennungseinrichtung (3) mit der Datenverarbeitungseinrichtung (24) und über diese mit wenigstens einem der folgenden Einrichtungen verbunden ist, bzw. diese in Abhängigkeit von der Augenstellung steuert: mechanische Mikroskopantriebe (23a), Mikroskopbeleuchtung (17), Patientendatenwiedergabeeinrichtung (22), Videoaufnahmegerät (26), Videorecorder (19), Bildliefervorrichtung für MRI-Bilddaten (20) oder Röntgen-Bilddaten (21).

8. Mikroskop nach einem der Ansprüche 2 bis 7, **dadurch gekennzeichnet, dass** der Datenverarbeitungseinrichtung (24) auch ein Schaltorgan - insbesondere ein Fussschalter (27) oder ein Akustikschalter - zugeordnet ist, um die Auswahl eines Befehles bzw. Befehlsymboles zu bestätigen.

9. Mikroskop nach einem der vorhergehenden Ansprüche, **dadurch gekennzeichnet, dass** die Pupillenpositionserkennungseinrichtung (3a,3b) teildurchlässige Spiegel (5a,b) umfasst, die für Licht im sichtbaren Bereich durchlässig, für IR-Licht jedoch reflektierend sind, und/oder dass einer Okularlinse (8a,8b) Einblendelemente (4a,b) vorgeschaltet sind, die IR-Licht einer IR-Quelle (13) auf ein Betrachterauge (1a,1b) lenken, wobei vom Betrachterauge reflektiertes IR-Licht einem CCD (6a,6b) - vorzugsweise durch Überlagerung der Mess- und Objektstrahlengänge dem CCD einer Videokamera (26) für die Aufnahme des Objekts - zugeführt ist, wobei im bevorzugten Fall eine Bildverarbeitungseinrichtung für das Auftrennen der Objektbildinformation und der IR-Bildinformation vorgesehen ist.

10. Mikroskop nach einem der vorhergehenden Ansprüche, **dadurch gekennzeichnet, dass** dem Tubus des Mikroskops wenigstens ein Trägerelement zugeordnet ist, auf der eine Markierung angeordnet ist, die für einen Betrachter durch die Okularlinse fixierbar ist, wobei die Pupillenpositionserkennungsvorrichtung (3) so ausgebildet ist, dass in Abhängigkeit von einer willkürlichen Augenstellung eine Eichroutine durchführbar ist, an Hand der eine Normalposition der Pupille festlegbar ist.

11. Mikroskop nach einem der vorhergehenden Ansprüche, **dadurch gekennzeichnet, dass** Bedienorgansymbole oder die in den Ansprüchen 2 oder 3 definierten Bedienorgansymbole an einer benutzeraktiven Displayoberfläche ausgebildet sind, der auch ein Zeigersymbol "Maus" zugeordnet ist, das durch - über die Pupillenpositionserkennungseinrichtung (3) bzw. über den Sensor verfolgte - Pupillenbewegung steuer- bzw. verschiebbar ist und dass mittels positioniertem Zeigersymbol die erwähnten oder zusätzliche Funktionen auslösbar sind.

12. Mikroskop nach einem der Ansprüche 2 bis 11, **dadurch gekennzeichnet, dass** die Datenverarbeitungseinrichtung (24) mit einer Datenaufbereitungseinheit (25) verbunden ist, die Daten aus Zusatzgeräten zu kompatiblen, schnell verarbeitbaren Daten aufbereitet bzw. EDV-Daten in geeignete Steuerdaten für die entsprechenden Bedienorgane, Geräte bzw. Vorrichtungen umwandelt.

13. Mikroskop nach einem der vorhergehenden Ansprüche, **dadurch gekennzeichnet, dass** im Okularbereich eine Positionierauflage für den Kopf bzw. das Gesicht eines Anwenders vorgesehen ist, die durch Fixierung von Gesichtspartien des Anwenders als horizontal und/oder Vertikalreferenz und/oder Entspannungshalterung dient.

## Claims

1. Microscope for a user, with at least one tube, at least one operating member, at least one remotely controlled actuating member and at least one controller for said actuating member and with a sensor for detecting the eye or pupil position of the eye of an observer for controlling the actuating member, **characterized in that** the actuating member comprises an auto zoom and/or drives for a zoom (15), for a focal adjustment means (16), for an eye distance adjustment apparatus, for an illumination (17) and/or a video recorder (19), an image supply apparatus for MRI image data (20) or X-ray data (21), and/or a patient data reproduction device (22), and **in that** for each eye (1a, 1b) of an observer, a pupil position detection device (3a, 3b) is provided which makes it possible to detect the position of each pupil from the associated beam path centre and is connected to an eye distance adjustment apparatus which automatically matches the tube distance to the observer's eye distance.

2. Microscope according to Claim 1, **characterized in that** in the region of the tube, means for displaying operating member symbols, which can be seen by the user inside the tube, are assigned to the eye (1), wherein a detection device is connected, via a data processing device (24) for detecting the field of vision, to the operating member symbols and further to the actuating member or to the drives for a zoom (15), for the focus adjustment means (16), for the eye distance adjustment apparatus and for the illumination means (17) and/or to the video recorder (19), to the image supply apparatus for MRI image data (20) or X-ray data (21), and/or to the patient data reproduction device (22).

3. Microscope according to Claim 1 or 2, **characterized in that** a display for superimposing switching symbols and/or a display for the displaying of operating member symbols, or of those operating member symbols which are defined in Claim 2, such that they can be seen by the user inside the tube is assigned to an eye (1), wherein, for the operating member symbols and for any MRI or X-ray or patient data, an LCD (2b or c) is preferably assigned as the display which images via an optical system (7c or d) and via a splitter mirror (10a or b) into an eye (1a, 1b) of the observer, wherein in the case of a stereomicroscope with two CCDs preferably one common background illumination (11) is assigned to the two CCDs.

4. Microscope according to one of the preceding claims, **characterized in that** the eye or pupil position detection device (3) or the sensor comprises at least one IR LED (13) and at least one CCD (6) for scanning the light of the IR LED (13) on the eye surface, wherein the CCD (6) is connected to an evaluation circuit or to a data processing device (24) or the data processing device which is defined in Claim 2, which controls the actuating member, or the drives for a zoom (15), for a focus adjustment means (16), for an eye distance adjustment apparatus, for an illumination means (17) and/or controls a video recorder (19), the image supply apparatus for MRI image data (20) or X-ray data (21), and/or the patient data reproduction device (22).

5. Microscope according to one of the preceding claims, **characterized in that** in the case of two sensors or two pupil position detection devices (3a, 3b), only one can optionally be operated, and/or **in that** modules which can be assembled in the manner of building blocks or can be switched on or off are assigned to the different functions.

6. Microscope according to one of Claims 2-5, **characterized in that** a memory for storing and automatically recalling adjustment data or processes of the microscope as a function of user codes, which can be input, is assigned to the data processing device (24).

7. Microscope according to one of Claims 2 to 6, **characterized in that** the pupil position detection device (3) is connected to the data processing device (24) and, by means of the latter, to at least one of the following devices, or controls them as a function of the eye position: mechanical microscope drives (23a), microscope illumination means (17), patient data reproduction device (22), video recording appliance (26), video recorder (19), image supply apparatus for MRI image data (20) or X-ray image data (21).

8. Microscope according to one of Claims 2 to 7, **characterized in that** a switching member - in particular a foot switch (27) or an acoustic switch - is also assigned to the data processing device (24) in order to confirm the selection of a command or command symbol.

9. Microscope according to one of the preceding claims, **characterized in that** the pupil position detection device (3a, 3b) comprises partially transparent mirrors (5a, b) which are transparent to light in the visible range but reflect IR light, and/or in that superimposition elements (4a, b) are connected upstream of an eyepiece lens (8a, 8b), which superimposition elements direct the IR light from an IR source (13) to the eye (1a, 1b) of an observer, wherein IR light reflected by the eye of the observer is supplied to a CCD (6a, 6b) - preferably by overlaying the measurement and object beam paths onto the CCD of a video camera (26) for recording the object -, wherein in the preferred case an image processing device for the splitting of the object image information and the IR image information is provided.

10. Microscope according to one of the preceding claims, **characterized in that** at least one carrier element is assigned to the tube of the microscope, on which carrier element a mark is arranged which, for an observer, can be fixed by way of the eyepiece lens, wherein the pupil position detection apparatus (3) is designed such that a calibration routine can be carried out as a function of an arbitrary eye position, which calibration routine is used to fix a normal position of the pupil.

11. Microscope according to one of the preceding claims, **characterized in that** operating member symbols, or the operating member symbols which are defined in Claim 2 or 3 are formed on a user-active display surface, to which a pointer symbol "mouse" is also assigned which can be controlled and displaced by way of pupil movement which is tracked via the pupil position detection device (3) or via the sensor, and **in that** the positioned pointer symbol is used to initiate the mentioned or additional functions.

12. Microscope according to one of Claims 2 to 11, **characterized in that** the data processing device (24) is connected to a data preparation unit (25) which prepares data from additional appliances to give compatible, quickly processible data or converts EDP data into suitable control data for the corresponding operating members, appliances or apparatuses.

13. Microscope according to one of the preceding claims, **characterized in that** in the eyepiece region, a positioning means for the head or the face of a user is provided which serves, by way of fixing facial parts of the user, as horizontal and/or vertical reference and/or relaxation holder.

## Revendications

1. Microscope destiné à un utilisateur, qui présente au moins un tube, au moins un organe d'entraînement, au moins un organe de réglage commandé à distance et au moins une entraînement pour cet organe de réglage, ainsi qu'un détecteur qui détecte la position des yeux ou des pupilles de l'oeil de l'observateur pour commander l'organe de réglage, **caractérisé en ce que** l'organe de réglage est un auto-zoom et/ou un entraînement pour un zoom (15), pour un réglage (16) de la focale, pour un dispositif de réglage de l'écartement entre les yeux, pour un éclairage (17) et/ou pour un enregistreur vidéo (19), un dispositif de transmission d'image qui transmet des données d'image MRI (20) ou des données de radiographie (21) et/ou un dispositif (22) de présentation de données concernant le patient, et **en ce qu'**un dispositif (3a, 3b) de détection de la position des pupilles est prévu pour chaque oeil (1a, 1b) de l'observateur pour rendre détectable la position de chaque pupille à partir du milieu du parcours de rayons qui y est associé et est relié à un dispositif de réglage de l'écart entre les yeux qui adapte automatiquement l'écart entre les tubes à l'écart entre les yeux de l'observateur.

2. Microscope selon la revendication 1, **caractérisé en ce que** dans la zone du tube, des moyens de représentation des symboles de l'organe d'entraînement visibles pour l'utilisateur à l'intérieur du tube sont associés à l'oeil (1), un dispositif de reconnaissance étant relié à un dispositif (24) de traitement de données pour détecter le recouvrement du regard et des symboles de l'organe d'entraînement et de plus à l'organe de réglage et aux entraînements pour un zoom (15), pour le réglage (16) de la mise au point, pour le dispositif de réglage de la distance entre les yeux, pour l'éclairage (17) et/ou à l'enregistreur vidéo (19), au dispositif de transmission d'image qui transmet les données d'image MRI (20) ou les données radiographiques (21) et/ou au dispositif (22) de présentation de données concernant le patient.

3. Microscope selon la revendication 1 ou 2,
**caractérisé en ce qu'**à au moins à un oeil (1) sont associés un affichage qui superpose les symboles de commutation et/ou un affichage qui représente de manière visible pour l'utilisateur à l'intérieur du tube des symboles des organes d'entraînement ou les symboles des organes d'entraînement définis à la revendication 2, un LCD (2b ou c) qui forme par une optique (7c ou d) et par un miroir diviseur (10a ou b) une image dans l'oeil (1a, 1b) de l'observateur étant utilisé comme affichage de préférence pour les symboles des organes d'entraînement ainsi que pour toutes les données MRI, radiographiques ou concernant le patient, tandis que dans le cas d'un microscope stéréo avec deux CCD, un éclairage commun de fond (11) est associé de préférence aux deux CCD.

4. Microscope selon l'une des revendications précédentes, **caractérisé en ce que** le dispositif (3) de reconnaissance de la position de l'oeil ou de la pupille et le détecteur comportent au moins une IR-LED (13) et au moins un CCD (6) pour l'exploration de la lumière de IR-LED (13) à la surface de l'oeil, le CCD (6) étant relié à un circuit d'évaluation et à un dispositif (24) de traitement de données ou au dispositif de traitement de données défini à la revendication 2 qui commandent l'organe de réglage et les entraînements pour un zoom (15), pour un réglage (16) de la distance focale, pour un dispositif de réglage de la distance entre les yeux, pour un éclairage (17) et/ou un enregistreur vidéo (19), le dispositif de transmission d'image qui transmet les données d'image MRI (20) ou les données radiographiques (21) et/ou le dispositif de présentation de données (22) concernant le patient.

5. Microscope selon l'une des revendications précédentes, **caractérisé en ce que** lorsque deux détecteurs ou deux dispositifs (3a, 3b) de reconnaissance de la position de la pupille sont prévus, un seul est mis sélectivement en fonctionnement et/ou **en ce qu'**aux diverses fonctions sont associés des modules qui peuvent être rassemblés en blocs et être raccordés ou débranchés.

6. Microscope selon l'une des revendications 2 à 5, **caractérisé en ce qu'**au dispositif (24) de traitement de données est associée une mémoire qui permet de conserver et d'appeler automatiquement les données et les opérations de réglage du microscope en fonction d'un code pouvant être introduit par l'utilisateur.

7. Microscope selon l'une des revendications 2 à 6, **caractérisé en ce que** dispositif (3) de reconnaissance de la position de la pupille est relié au dispositif (24) de traitement de données et par celui-ci à au moins l'un des dispositifs qui suivent, pour les commander en fonction de la position de l'oeil : entraînements mécaniques (23a) du microscope, éclairage (17) du microscope, dispositif (22) de présentation de données du patient, appareil (26) d'enregistrement vidéo, enregistreur vidéo (19), dispositif (20) de transmission des données d'image MRI ou des données d'image radiographiques (21).

8. Microscope selon l'une des revendications 2 à 7, **caractérisé en ce qu'**au dispositif (24) de traitement de données est également associé un organe de commutation, en particulier un commutateur à pédale (27) ou un commutateur acoustique, pour sélectionner d'un ordre ou confirmer le symbole d'un ordre.

9. Microscope selon l'une des revendications précédentes, **caractérisé en ce que** le dispositif de reconnaissance (3a, 3b) de la position de la pupille comporte des miroirs (5a, 5b) partiellement transparents qui sont transparents à la lumière visible mais qui réfléchissent la lumière infrarouge et/ou **en ce que** des éléments de superposition (4a, 4b) placés devant une lentille d'oculaire (8a, 8b) dévient la lumière IR d'une source IR (13) vers l'oeil (1a, 1b) de l'observateur, la lumière IR réfléchie par l'oeil de l'observateur étant conduite à un CCD (6a, 6b), de préférence par superposition des trajets des rayons de mesure et d'objet au CCD d'une caméra vidéo (26) qui enregistre l'objet, et dans le cas préféré, un dispositif de traitement d'image est prévu pour séparer les informations d'image de l'objet et les informations d'image dans l'infrarouge.

10. Microscope selon l'une des revendications précédentes, **caractérisé en ce qu'**au tube du microscope est associé au moins un élément de support sur lequel est agencé un repère qui peut être fixé pour un observateur par la lentille d'oculaire, le dispositif (3) de reconnaissance de la position de la pupille étant configuré de façon qu'une routine d'étalonnage puisse être accomplie en fonction d'une position arbitraire de l'oeil et permet de définir la position normale de la pupille.

11. Microscope selon l'une des revendications précédentes, **caractérisé en ce que** des symboles des organes d'entraînement ou les symboles des organes d'entraînement définis dans les revendications 2 ou 3 sont présentés à l'utilisateur sur une surface d'affichage active, à laquelle est également associé un symbole indiquant "souris" qui peut être commandé ou déplacé par un déplacement de la pupille suivi par le dispositif (3) de reconnaissance de la position de la pupille ou par le détecteur et **en ce que** les fonctions mentionnées ou des fonctions supplémentaires peuvent être déclenchées en positionnant le symbole indicateur.

12. Microscope selon l'une des revendications 2 à 11, **caractérisé en ce que** le dispositif (24) de traitement de données est relié à une unité (25) de traitement de données qui transforme les données d'appareils supplémentaires en données compatibles rapides à traiter ou convertit des données d'ordinateur en données de commande appropriées pour les organes de commande, appareils ou dispositifs correspondants.

13. Microscope selon l'une des revendications précédentes, **caractérisé en ce que** dans la zone de l'oculaire est prévu un appui de positionnement pour la tête ou le visage de l'utilisateur qui sert de référence horizontale et/ou verticale et/ou de support de détente en immobilisant des parties du visage de l'utilisateur.
